# NEW EUROPEAN PATENT SPECIFICATION

(11) **EP 2 404 584 B2**
(45) Date of publication and mention of the opposition decision: **27.09.2023**
(45) Mention of the grant of the patent: 08.07.2020
(21) Application number: 10169007.1
(22) Date of filing: 09.07.2010
(51) Int. Cl.: A61F 13/20

(54) **TAMPON DISPOSED WITH LONGITUDINAL GROOVES AND INDENTATIONS**
Mit Längsrillen und -einkerbungen versehenes Tampon
Tampon disposé avec des rainures et indentations longitudinales

(43) Date of publication of application: 11.01.2012
(73) Proprietor: Ontex Hygieneartikel Deutschland GmbH, 02692 Grosspostwitz (DE)
(72) Inventor: SMET, Steven, 9240, Zele (BE)
(74) Representative: Saurat, Thibault

(56) References cited:
- EP-A1- 2 448 536
- EP-B1- 1 383 453
- WO-A1-2009/129910
- WO-A1-2009/129910
- WO-A1-2010/069908
- WO-A2-2010/055115
- DE-A1-102005 050 514
- US-A- 2 798 260
- US-A- 4 175 561
- US-A- 5 592 725
- US-A1- 2005 113 780
- US-A1- 2005 177 090
- US-A1- 2007 083 182
- US-A1- 2010 121 251

## Description

### FIELD OF THE INVENTION

The invention concerns a tampon, in particular for feminine hygiene, disposed with indentations exclusively between the longitudinal grooves.

### BACKGROUND TO THE INVENTION

From the prior art, cylindrical shaped tampons are known having ribs defined by longitudinal grooves, as described, for example, in WO 02/078586, EP 0 422 660, US 2002/0157222, US 5,592,725, US 5,895,408, EP 1 108 408, US 2003/0208180, WO 00/53141, EP 0 639 363 US2007083182, EP2448536, US2010121251 and WO 2009/129910.

US 2005/0177090 A1 discloses a tampon for feminine hygiene that has an insertion end, a withdrawal end, a central portion between the insertion end and the withdrawal end, a length, a longitudinal axis, a radial axis, and an outer surface. The tampon is made of compressed fibrous material. The outer surface of the insertion end has a plurality of longitudinal grooves which are offset from the plurality of longitudinal grooves located in the withdrawal end. In a particular embodiment, the central portions may comprise surface aberrations including protuberances, depressions and mixtures thereof.

US 4,175,561 A discloses feminine hygienic pads with improved absorption properties for use in the interlabial space in the area of the female urethral meatus and vaginal meatus for absorbing urine and other undesirable exudations comprising an elongated fibrous pad base of cylindrical, elliptical, polygonal or similar regular shape, the surface of the pad base being formed with multiple flutings, a multiplicity of absorptive cells or pin-cushioning or being formed wholly or in part of shaggy material to increase the absorptive surface area of the pad. US 4,175,561 A further discloses methods of forming elongated fibrous feminine hygienic pads for interlabial use comprising the steps of applying flutings, absorptive cells or pin-cushioning to either continuous or discontinuous strands of absorptive material travelling in one direction, the flutings or the like being applied by fingers moving back and forth at right angles to the direction of travel of the strand and compacting the fibrous material at the points of contact, the movement of the strand being momentarily arrested as the fingers contact the strand, and cutting off segments.

Tampons of the prior art by the nature of the design tend to have a limited absorbent and expansion capacity.

Furthermore, prior to insertion into the body cavity and during use, the tampons of the prior art can feel uncomfortable.

There is a need for a new design of tampon, a device for its manufacture and a method therefore which overcomes the problems of the prior art.

### AIMS OF THE INVENTION

The object of the invention is to provide a new type of tampon with indentations in the space between the longitudinal grooves that convey liquid directly to the tampon core leading to faster absorption and increased availability for absorption by the longitudinal grooves.

The advantages will become clear to the persons skilled in the art from the description and the accompanying figures provided below.

### BRIEF DESCRIPTION OF THE FIGURES

**FIGs. 1A** to **1C** depict numerous views of a finished tampon 220 disposed with a plurality of chevron-shaped indentations.
**FIGs. 2A** to **2C** depict numerous views of a finished tampon 220 disposed with a plurality of circular indentations.
**FIGs. 3A** to **3C** depict numerous views of a finished tampon 220 disposed with a plurality of cross-shaped indentations.
**FIG. 4** shows in schematic form an arrangement of press jaws according to an embodiment of the invention, viewed along the press axis, where the jaws comprise alternately penetrating segments for the longitudinal groove and for the indentations.
**FIGs. 5** to **11** depict a pressing cycle, FIG. 5, tampon blank is inserted into a press opening; FIG. 6, the jaws advance; FIG. 7 the jaws reach the closed position. FIG. 8 the jaws partially retract to a holding position; FIG. 9 the pressed blank tampon is slidably ejected; FIG. 10 the press jaws retract to the open position; FIG. 11 a new tampon blank is inserted into the press opening.
**FIG. 12** shows a three dimensional representation of a mechanism for control movement of the press jaws.
**FIGs. 13A to 13C** depict a press jaw disposed with a penetrating segment for a longitudinal groove in side (FIG. 13A), front (FIG. 13B) and plan (FIG. 13C) views.
**FIGs. 14A** to **14C** depict a press jaw disposed with penetrating segments for indentations in side (FIG. 14A), front (FIG. 14B) and plan (FIG. 14C) views.
**FIG. 15A** shows the plan view of the pressing end a press jaw 6, provided with a plurality of chevron shaped penetrating segments for indentations.
**FIGs. 15B** to **15D** depict numerous views of a finished tampon **220** pressed using a press comprising the press jaw depicted in **FIG. 15A****.**

### SUMMARY OF SOME EMBODIMENTS OF THE INVENTION

One embodiment of the invention relates to a tampon (200) for feminine hygiene having a longitudinal body showing in compressed condition a length and a width, whereby said tampon has the features of claim 1.

Another embodiment of the invention relates to a tampon (200) as described above, having a mushroom-shape, domed head, constricted withdrawal end, conical withdrawal end, barrel shape, rivet shape, finger recess, withdrawal cord or a bullet shape.

Another embodiment of the invention relates to a tampon (200) as described above, disposed in an applicator.

Another embodiment of the invention relates to a tampon (200) as described above, wherein said longitudinal ribs are at least partially relatively uncompressed compared with the fibre core.

Another embodiment of the invention relates to a tampon (200) as described above, provided with one or more markings on the surface.

Another embodiment of the invention relates to a tampon (200) as described above, provided with one or more markings on the surface.

Another embodiment of the invention relates to a tampon (200) as described above, wherein at least one indentation (224) is spatially separated from other adjacent indentations.

Another embodiment of the invention relates to a press (100) for manufacturing a tampon described herein, having the features of claim 7.

Another embodiment of the invention relates to a press (100) described above, wherein the press jaws are configured to move synchronously.

Another embodiment of the invention relates to a press (100) described above, wherein at least one, preferably each and every PSI is in the shape of a chevron (v-shaped), straight-edged slot, undulating, star, cross, diamond, circular, oval, triangle, rectangle, pentagon, sexagon, septagon, octagon, nonagon, decagon, or other polygon.

Another embodiment of the invention relates to a press (100) described above, wherein the number of PSSGs disposed on a press jaw is between 3 and 7.

Another embodiment of the invention relates to a press (100) described above, wherein the maximum height, DL, of the PSLG from the base (17) to the tip (15) is greater than the maximum height, DS, of the PSI from the base (17) to the tip (15).

Another embodiment of the invention relates to a press (100) described above, wherein the press jaws further comprise one or more pressing shoulders for finish shaping of the preform.

Another embodiment of the invention relates to a press (100) described above, wherein during pressing, the pressing shoulders are configured to produce a preform having a mushroom-shape, domed head, constricted withdrawal end, conical withdrawal end, barrel shape, rivet or a bullet shape.

Another embodiment of the invention relates to a press (100) described above, wherein:
- each press jaw (6) is connected to a longitudinal transmission rod (50), aligned essentially radially to the press axis (4), or inclined to the radius centred on the press axis, said rod having a proximal end (56) closer to the press axis (4) and, at the opposing longitudinal side, a distal end (54) directed away from the press axis (4), and is configured for slidable linear displacement along an axis of movement (52) that is essentially radial to the press axis (4), or inclined to said radius.
- the press further comprises a rotatable annular plate (64) having a central axis in co-axial alignment with press axis (4) and provided with a plurality of discrete slots (66) on the plate (64), one slot for each rod, which slot engages with a roller (68) in revolute attachment to the distal end (54) of each rod (50), the roller (68) being in slidable connection with the slot (66), the axis of rotation of the roller (68) being perpendicular to the longitudinal axis of the rod (52) and is parallel with the press axis (4),
- the ring (60) is configured to rotate and thereby effect movement of the roller (68) and translation the rod (50) towards or away from the press axis (4) along the axis of movement (52), and
- the slot (66) shaped to retract or advance each press jaw (6) in the direction of the press axis (4) according to the angle of rotation of the annular plate (64) around its central axis.

Another embodiment of the invention relates to a press (100) described above, wherein the press jaws (6) in step b) are moved to a closed position in a direction essentially radial or inclined to the radius of the press axis (4).

Another embodiment of the invention relates to a press (100) described above, wherein the press jaws (6) in step c) are moved to a holding position between the closed position and open position, in which the tips (15') of the PSIs (11) are retracted to a greater distance from the press axis (4) compared with the tips (15) of the PSLGs, such that the PSIs are fully withdrawn from the preform, while contact is maintained between the preform longitudinal grooves and at least one PSLG (13).

Another embodiment of the invention relates to a press (100) described above, wherein at least one indentation is spatially separated from other adjacent indentations.

Another embodiment of the invention relates to a process for manufacturing a tampon described herein having a longitudinal axis, comprising the features of claim 17.

Another embodiment of the invention relates to a process as described above, wherein the press is as defined above.

### DETAILED DESCRIPTION OF THE INVENTION

Unless defined otherwise, all technical and scientific terms used herein have the same meaning as is commonly understood by one of skill in the art.

The recitation of numerical ranges by endpoints includes all integer numbers and, where appropriate, fractions subsumed within that range (e.g. 1 to 5 can include 1, 2, 3, 4 when referring to, for example, a number of articles, and can also include 1.5, 2, 2.75 and 3.80, when referring to, for example, measurements). The recitation of end points also includes the end point values themselves (*e.g*. from 1.0 to 5.0 includes both 1.0 and 5.0).

In the following detailed description of the preferred embodiments, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

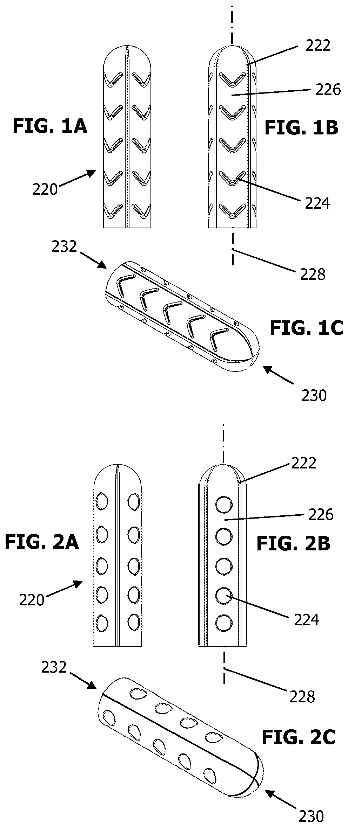

With references to **FIGs. 1A** to **3C****,** present invention relates to a tampon **220** in particular for feminine hygiene, having a longitudinal body in an essentially cylindrical shape. The tampon comprises compressed absorbent fibrous material and has an outer circumferential surface which is divided into a number of longitudinal grooves **222** that flank longitudinal ribs, and is provided with a plurality of indentations **224** that are arranged in the longitudinal direction, flanked by two longitudinal grooves *i.e.* along a rib. At least one, preferably each and every indentation **224** is in spatial isolation from one or both flanking longitudinal grooves **222**. The at least one, preferably each and every indentation **224** may also be spatially separated from other or adjacent indentations **224**. In other words, at least one, preferably each and every indentation **224** may be in spatial isolation from neighbouring indentations **224** and/or from neighbouring longitudinal grooves **222**. The indentations are sometimes known as side grooves insofar as they are disposed at the lateral sides of the longitudinal grooves.

The tampon **220** is at least partially provided with longitudinal ribs **226** defined by longitudinal grooves **222**. The longitudinal ribs **226** are straight and preferably parallel to the longitudinal axis of the tampon, however, they may, alternatively, be straight and inclined to the longitudinal axis of the tampon. The number of longitudinal ribs **226** can vary, for example depending on the diameter of the tampon and/or the type of absorption material. Preferably, there are between 4 and 12 ribs, more preferably there are between 6 and 12 ribs and even more preferably, at least four. While the present invention, like many known tampons, may have an even number of ribs, it is also within the scope of the present invention to produce tampons with an odd number of ribs. The number of indentations **224** can vary, for example, depending on the length of the tampon and/or the type of absorption material. The number of indentations may be equal to or at least 2, 3, 4, 5, 6, 7, 8, 9, 10, or 12 for each longitudinal groove or rib. Preferably, there are between 3 and 10 indentations for each longitudinal groove **222**, more preferably between 4 and 7, most preferably 4 or 5. At least one rib, preferably each and every rib is provided with side grooves.

The tampon **220** generally has a compressed, central, solid, generally cylindrical fiber core with a high degree of compression, which ensures the stability or column strength of the tampon during digital introduction of the tampon into a body cavity. The longitudinal ribs disposed with indentations are relatively less compressed than the core and have, in particular on a circumferential surface of the tampon, a softer fibrous structure. The longitudinal ribs extend essentially radially outward at equal circumferential angle intervals from this solid fiber core.

In a more preferred embodiment, the longitudinal grooves **222** are closed, at least at the circumferential surface of the tampon, as the side flanks of adjacent longitudinal ribs touch one another to form the soft, closed circumferential surface of the tampon. This circumferential surface of the tampon makes possible more gentle and, therefore, more pleasant introduction of the tampon into the body cavity.

The indentations **224** in the tampon form depressions on the tampon surface, suitable for channelling liquid into the tampon **220**. Absorbency of the tampon **220** is, therefore, improved, because the surface is more open. After the introduction of the tampon into the body cavity, these indentations **224** convey the body fluid directly to the fiber core, in order to utilize its fibrous material immediately to increase the absorption capacity and expansion capacity of the tampon and to accelerate the opening of the closed longitudinal grooves **222** outward. Separating the indentations from the longitudinal grooves spatially, ensures a flow of liquid directly into the core rather than along the longitudinal groove. Therefore, the arrangement of the indentations **224** and longitudinal grooves **222** brings about an enlargement of the surface of the tampon **220** which results in the absorption capacity and expansion capacity of the fiber core being improved considerably. Further, there is a more rapid take-up of body fluid. At the same time, a reduction in the weight of fibrous material used in the tampon is thus possible, which allows more economical production of the tampon.

According to a further embodiment of the invention, the circumferential surface of the tampon and its fiber core can also be substantially cylindrical with a circular cross-section, or even an oval cross-section.

Absorbent fibrous material usable in the tampon according to the invention may consist of any absorbent material having acceptable absorbency and modulus of elasticity properties that is capable of absorbing and/or retaining liquid. The absorbent structure can be manufactured in a wide variety of sizes and shapes and from a wide variety of liquid-absorbing materials. It is, of course, desirable to use absorbent materials having a minimum content of extraneous soluble materials since the product may be retained in the body for a considerable period of time, *i.e.* absorbent materials contain no/little unnecessary soluble matter which could dissolve and enter the body. Retained soluble extraneous materials could cause a safety hazard if they are toxic, irritant, or sensitive. A representative, non-limiting list of useful materials includes cellulosic materials, such as rayon, cotton, wood pulp, creped cellulose wadding, tissue wraps and laminates, peat moss, and chemically stiffened, modified, or cross-linked cellulosic fibres; synthetic materials, such as polyester fibres, polyolefin fibres, absorbent foams, *e.g*. a flexible resilient polyurethane foam, absorbent sponges, super-absorbent polymers, absorbent gelling materials; formed fibres, such as capillary channel fibres and multi limbed fibres; synthetic fibres, or any equivalent material or combinations of materials, or mixtures of these.

In one embodiment, the invention provides a tampon, wherein said tampon is not covered. Preferably, tampons without covering are made from pure cotton.

In a preferred embodiment, the invention provides a tampon, wherein said tampon is at least partially surrounded by a covering. The covering is preferably not provided over the insertion end, in order to provide better access of the menses to the insertion end of the tampon. In order to improve the absorbing capacity and expansion capacity of the tampon, said covering is preferably a stretchable or elastic liquid-permeable covering. The covering can consist of, for example, a non-woven covering material made of, for example, thermoplastic, heat sealing fibers or a plastic film. Such a covering improves the comfort of introduction and prevents fibres being detached during introduction or removal of the tampon into or from the body cavity.

A further preferred feature of the tampon of the invention is a withdrawal cord, extending from the withdrawal end of the tampon, in order to ease withdrawal of the tampon.

Also, the tampon is preferably provided with a round domed insertion end of high compression. This will make insertion of the tampon easier because the narrowed end goes deepest in the vagina.

Another embodiment of the present invention is a tampon as described above, in which the tampon is mushroom shaped. Another embodiment of the present invention is a tampon as described above, in which the tampon is rivet shaped. Another embodiment of the present invention is a tampon as described above, in which the tampon is bullet shaped. Another embodiment of the present invention is a tampon as described above, in which the tampon is barrel shaped.

A tampon may further be provided with a constricted, preferably conical withdrawal end. The conical shape is one which is preferably truncated from its point. Such conical end guides the tampon during withdrawal, so making withdrawal easier.

In a further preferred embodiment, the withdrawal end is provided with a finger recess according to any technique known in the art. This facilitates the handling and the insertion of the tampon.

The tampon has a round domed insertion end, to facilitate insertion of the tampon, and is further provided with a withdrawal cord at the withdrawal end in order to facilitate withdrawal of the tampon after use.

Furthermore, the present invention relates to tampons, which can be applied digitally, as well as to tampons that can be applied with an applicator. An applicator used to position the tampon within the vagina can be any applicator known to those skilled in the art, e.g. the telescoping tube type applicator. The applicator can be made of any of the acceptable materials, e.g. cardboard or molded polyethylene. The applicator can be sized similarly to those presently commercially used.

A tampon of the invention may optionally be provided with one or more markings on the surface. A marking may be provided by any mean means including printed using inks, or by impression. A marking may comprise any features including alpha numerals, graphic illustrations, patterns and/or photographic illustration. A marking may be, for example, information such as expiry date, absorbent capacity, use instruction, warning indications. Where a tampon is provided with information, it is an information carrier. A marking may also be advertising. A marking may provide product appeal to the user or groups of users. For example, it may comprise image, pattern, graphic or alpha numeral designed to appeal to a mind set of a user group by way of aesthetic appearance and/or life-style association (e.g. cartoons, logos etc.).

A tampon of the invention may optionally be provided in one or more colours. Colours may be printed as mentioned above, or impregnated into the material. A colour may indicate an expiry date, an absorbent capacity, a size or other information regarding the product. A colour may be designed to appeal to a mind set of a user group by way of aesthetic appearance and/or life-style association.

It is a further aspect of the invention that a tampon is provided with chemical indicator that is capable of indicative colour change. Such indicator may show, for example, a medical condition. The chemical indicator may react within one or more agents in bodily fluids to indicate an abnormality. For example, a chemical indicator may change colour when a subject is suffering such as anaemia (by detecting iron/haemoglobin density), diabetes (by detecting glucose), position in the menstrual cycle (by detecting hormones), the presence of sexually transmitted diseases (by detecting antigens towards for example, gonorrhea, syphilis, hepatitis A, B or C, herpes, HIV, chlamydia) etc.

The indentations may have any suitable profile, for example, essentially of a chevron (v-shaped), straight-edged slot, undulating, star, cross, diamond, circular, oval, triangle, rectangle, pentagon, sexagon, septagon, octagon, nonagon, decagon, other polygon, or the like. Each and every indentation may have the same profile, or at least two indentations may have different profiles. Profile refers to the shape of the indentation as observed on the surface of the tampon. At least one, preferably all the indentations may be spatially separated from the flanking longitudinal grooves, in which case the at least one, preferably all the indentations will be present exclusively in the longitudinal ribbed part of the tampon. By spatially separated means that the outer profile of an indentation on the surface of the tampon does not touch the outer profile of a neighbouring indentation and/or of a neighbouring longitudinal groove on the surface of the tampon. Each indentation may project towards the core of the tampon, but may not enter the core. Examples of different configurations of the indentations are given in **FIGS. 1A** to **3C****.**

**FIGs. 1A** to **1C** depict numerous views of a finished tampon **220** according to the invention having a rounded insertion end **230** and a withdrawal end **232**. It has four longitudinal grooves **222**, and a plurality of chevron shaped indentations **224** spatially separated in the longitudinal direction **226** of the tampon **220**. The indentations are in spatial isolation from neighbouring indentations *i.e.* they do not touch other indentations or flanking longitudinal grooves.

**FIGs. 2A** to **2C** depict numerous view of a finished tampon **220** according to the invention having a rounded insertion end **230** and a withdrawal end **232**. It has four longitudinal grooves **222**, and a plurality of circular indentations **224** spatially separated in the longitudinal direction **226** of the tampon **220**. The indentations are in spatial isolation from neighbouring indentations *i.e.* they do not touch other side or flanking longitudinal grooves.

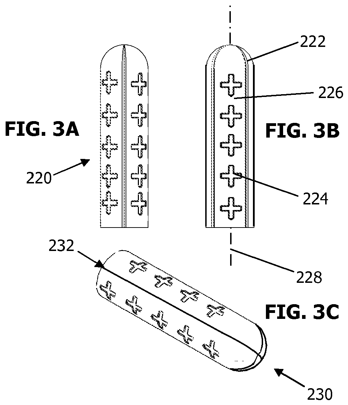

**FIGs. 3A** to **3C** depict numerous view of a finished tampon **220** according to the invention having a rounded insertion end **230** and a withdrawal end **232**. It has four longitudinal grooves **220**, and a plurality of cross-shaped indentations **222** spatially separated in the longitudinal direction of the tampon **226**. Each cross is orientated such that the two opposing corners of the cross are aligned with the longitudinal axis of the tampon. The indentations are in spatial isolation from neighbouring indentations *i.e*. they do not touch other indentations or flanking longitudinal grooves.

The present invention also concerns an apparatus, specifically a press, for manufacturing the tampon described above. In the prior art, pressing machines have penetrating segments, which form longitudinal ribs defined by longitudinal grooves and which penetrate the absorbing material. Such machines are known for example from EP 0 422 660, EP 0 639 363 and WO 02/078586.

The apparatus of the current invention comprises a press having press jaws each having a pressing end and opposite thereto a back end, which jaws are arranged in a star formation with respect to the press axis and preferably, but not necessarily, at the same radial distance from the press axis at least in the open position. The pressing end of the jaws are directed towards the press axis. The jaws can be moved (*i.e*. advanced and/or retracted) in a common plane, towards the press axis between an open position, holding position and closed position and, in their closed position, are preferably supported on one another on their mutually opposite longitudinal sides. The movement towards the press axis may be radial or inclined with the radius of the press axis.

The jaws preferably move synchronously to provide an enhanced geometric uniformity of the longitudinal grooves, indentations and ribs. Accordingly, the integrity of the non-woven material surrounding the tampon blank is maintained. Tension is typically formed across a rib in the non-woven cover because the cover is stretched by its folding into the longitudinal grooves. The pressing of indentations into the ribs would normally cause the cover to tear. However, by simultaneously pressing the longitudinal grooves and indentations, tensions in the cover are evenly distributed . Preferably , the tampon blank is compressed such that said longitudinal ribs extend outward at equal circumferential angle intervals.

A preferred press consists of at least 3 press jaws, preferably 8 (*e.g.* 4 PSLG + 4 PSI) press jaws. It is desirable to equip the press with an even number of press jaws (*e.g.* 2, 4, 6, 8), but other numbers of press jaws can be used, including odd numbers (*e.g.* or 3, 5, 7, 9). The number of press jaws can vary, for example depending on the weight and the composition of the material intended for the tampon and can also be smaller or greater than eight, although the number generally should not be under three. One or more penetrating segments for pressing the grooves and indentations are provided at the pressing end of the jaw. Arranged on the jaws at the pressing end are penetrating segment for pressing the longitudinal groove (PSLG) and penetrating segments for pressing the indentations (PSI).

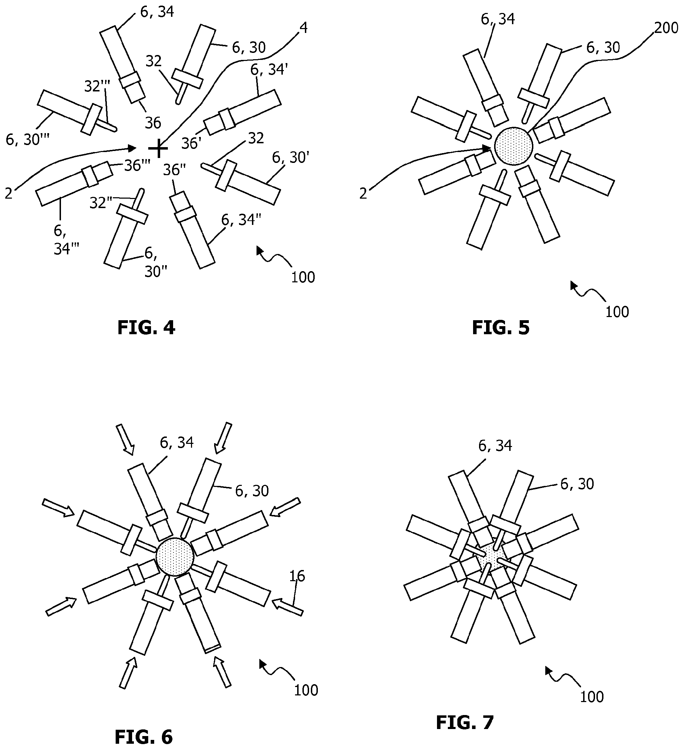

The press jaws may be arranged circumferentially in adjacent pairs, one jaw of the pair comprising at the pressing end a penetrating segment for pressing the longitudinal groove (PSLG) and the second jaw of the pair comprising at the pressing end penetrating segments for pressing the indentations (PSI). As such, the circumferential arrangement of jaws alternates (see **FIGs. 4** to **11**). The number of jaws in the press disposed with the PSLG is equal to the number disposed with the PSI, which may be equal to or at least 2, 3, 4 pairs of press jaws.

Alternatively, the PSLG and PSI may be disposed on the same, single press jaw, the PSI arranged on either or both longitudinal sides of the PSLG. When the PSLG and PSI are provided on the same press jaw, the number of jaws in the press may be equal to or at least 3, 4, 5, 6, 7, or 8. It is within the scope of the invention that the PSIs are disposed on a plurality (e.g. at least 1, 2, 3, 4, 5, 6, 7, 8) of adjacent press jaws, which combine to form a larger PSI that spans a larger circumferential arc than would be possible with a single press jaw.

It will be appreciated that pressing shoulders may be provided as separate press jaws, for example, alternating with jaws having penetrating segments. According to one aspect of the invention, the press jaws may be arranged circumferentially in adjacent pairs, one jaw of the pair comprising at the pressing end the PSLG and PSI and the second jaw of the pair comprising at the pressing end a pressing shoulder. When the PSLG and PSI are provided on the same press jaw, the number of jaws in the press including jaws containing the pressing shoulder may be equal to or at least 3, 4, 5, 6, 7, or 8. In an alternative arrangement, the pressing shoulder may be combined with press jaws disposed with penetrating segments. For instance, a press jaw may have a pressing shoulder provided with penetrating segments for pressing the indentations (PSI). In the alternative, a press jaw may have a combination of one PSLG and a pressing shoulder arranged either or both sides of the PSLG, the pressing shoulder optionally provided with the PSI. It is well understood in the art that the pressing shoulder is a shaping tool for finish shaping of the preform, that applies radial pressure on the circumferential surface of the ribs of the perform subsequent to impression of the tampon by the penetrating segments. The pressing shoulders can be straight or angular, but preferably have a curvature in the transversal direction in order to press the circumferential surface of the tampon blank into an essentially cylindrical form of smaller diameter. A pressing shoulder may contain one or more slots to accommodate the PSIs of a neighbouring jaw.

It is an aspect of the invention that the press jaws can be moved into an open, closed and a holding position that is between the closed and open position. In the open position, the opening formed in the press is of sufficient size for insertion of a tampon blank. In the closed position, the tampon blank is pressed. In the holding position, the preform is suspended on the tips of at least two penetrating segments, and can be removed (ejected) from the press, preferably slidably, without significant damage. By arranging a slidable ejection while the tampon is suspended by the tips of the longitudinal penetrating segments, the integrity of the tampon is maintained, avoiding damage to its surface by otherwise protruding PSIs in the ejection passage. Equally, ejection of the tampon while the jaws are open fully would lead to damage as the space between two jaws into which the tampon is released provides only a obstructive and unhygienic passage for slidable ejection.

According to one aspect of the invention, the PSLG and PSI retract differentially subsequent to pressing the preform. The PSI retracts to a greater extent than the PSLG after pressing and prior to ejection of the preform so formed. In other words, the press jaws are retracted to the holding position between the closed position and the open position of the jaws, in which the tips of the PSIs are moved (retracted) to a greater distance from the press axis compared with the tips of the PSLGs, such that the PSIs are withdrawn from the preform, while contact is substantially maintained between the preform longitudinal grooves and the PSLGs. The PSIs may be fully withdrawn from the preform.

The differential retraction allows removal (ejection) of the tampon longitudinally while the jaw is still partially closed. At the moment of ejection, the press jaws open partially to a holding position in which the PSIs are fully withdrawn and clear of the preform, while the PSLGs maintain contact with the tampon longitudinal grooves. Thus, the tampon is suspended in the partially closed jaws by the circumferential arrangement of PSLGs, but is free from contact with the PSIs. Accordingly, the tampon can be propelled and ejected longitudinally while in slidable contact only with the star-shaped arrangement of PSLGs. By arranging a slidable ejection along the PSLGs, the integrity of the tampon is maintained, avoiding damage to its surface by otherwise protruding PSIs in the ejection passage.

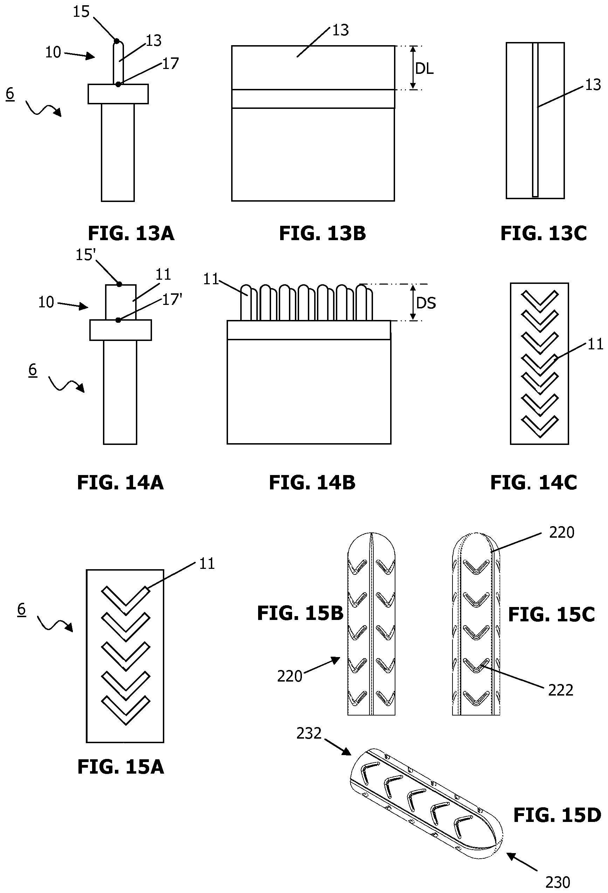

Differential retraction of the penetrating segments may be achieved by differential lengths of the respective PSLG and PSI *i.e.* the PSLG is longer than the PSI. This is illustrated in **FIGs 13B to 14B**, where the height, DL, of the PSLG from the base **17** to the tip **15** is greater than the height, DS, of the PSI from the base **17'** to the tip **15'.** Accordingly, the jaws may be configured to retract synchronously by an equal distance until the holding position is maintained. Alternatively, differential retraction may be achieved by different radial press jaw lengths or by retracting the respective press jaws by different amounts; this applies when the PSLG and PSI are disposed on separate press jaws.

One embodiment of the invention is a press **100** for manufacturing a tampon, comprising at least three press jaws **6** arranged in a star around a central longitudinal press axis **4** forming a press opening **2**, whereby there is provided on a single or separate adjacent press jaws **6**:
- a penetrating segment, PSLG, **13** configured to penetrate the absorbing material with a longitudinal groove, and
- penetrating segments, PSIs, **11** configured to penetrate the absorbing material with a plurality of indentations that are arranged in the longitudinal direction,
wherein the press is configured, preferably sequentially, to:
a) load a cylindrical blank **200** in the press opening **2**,
b) move the press jaws **6** to a closed position to press the cylindrical blank **200** so forming a preform **210**,
c) move the press jaws **6** to a holding position between the closed position and open position, so that the preform can be removed,
d) remove the preform while the jaws (6) are in the holding position, and
e) move the press jaws **6** to the open position for loading of a subsequent cylindrical blank.

At least one, preferably all the PSIs arranged in the longitudinal direction on a press jaw, are in spatial isolation from neighbouring PSLGs and/or other PSIs. In step b) the press jaws **6** is moved (advanced) to a closed position to press the cylindrical blank **200**, so forming a preform **210**. The movement, towards the press axis, is preferably radial or may be inclined to the radius of the press axis. The cylindrical blank is, thus, pressed radially or may be pressed inclined to the radius of the press axis.
In step c), the press jaws **6** are moved (retracted) to a holding position between the closed position and open position, so that the preform can be removed axially without substantial obstruction. Preferably, the holding position is where the tips **15'** of the PSIs **11** are moved (retracted) to a greater distance from the press axis **4** compared with the tips **15** of the PSLGs, such that the PSIs are withdrawn, preferably fully, from the preform, while contact is maintained between the preform longitudinal grooves and the PSLGs **13**.
In step d) the preform is axially removed (ejected), preferably slidably, while the jaws (6) are in the holding position,
In step e) the press jaws **6** are moved (retracted) to the open position for loading of the next cylindrical blank. The sequence a) to e) may be repeated for a subsequent cylindrical blank.

The penetrating segment for pressing the longitudinal groove (PSLG) is configured to press essentially longitudinal grooves into the blank tampon. It is provided at the pressing end of the press jaw. The longitudinal grooves extend fully from the insertion end to the withdrawal end, and are preferably parallel to the press axis. The PSLG, is a straight, blunt blade having a tip oriented towards the press opening. It is longitudinal and preferably the longitudinal length is aligned with the press axis, but may equally be inclined to the press axis. Preferably, when the press jaw is closed, each PSLG is orientated in the cutting direction radially or inclined to the radius of the press axis, thereby giving rise to radial or radially-inclined grooves in the tampon. The height, DL, of the PSLG from its base to the tip (FIG. 13B) may be 10%, 20 %, 30 %, 40 %, 50 %, 60 %, 70 %, 80 %, or 90 % greater than the height, DS, of the PSI (FIG. 14B), or greater by a value in the range between any two of the aforementioned values. Preferably, the height of the PSLG is essentially constant along the longitudinal length of the press jaw.

With reference to **FIGs. 13A** to **C** which depict a press jaw **6** in side (**13A**), front (**13B**) and plan (**13C**) views, the PSLG **13** is provided on the pressing end of the press jaw **6** and is an essentially planar oblong structure protruding from the base of the pressing end. According to one aspect of the invention, the transverse profile of the PSLG may be symmetrical along its length (from base **17** to tip **15**) as is shown, for example, in **FIGs. 13A**, reference sign **13**. Alternatively, the penetrating segment may be asymmetric along its length, wherein one edge of the penetrating segment is straight and the other curved.

The penetrating segments for pressing the indentation (PSIs), are configured to press a plurality of indentations into the blank, which indentations are arranged in the longitudinal direction. At least one, preferably all the PSIs are spatially separated (*i.e.* isolated or do not touch) from each other in the longitudinal direction and/or from neighbouring longitudinal grooves. At least one, preferably all indentations may be provided exclusively on a rib flanked by two longitudinal grooves, *i.e.* they may not adjoin or cross a groove. The indentations are spatially arranged in the longitudinal direction. Each and every indentation along a rib may have the same profile (*e.g.* all chevron), or at least two indentations may have different profiles (e.g. (*e.g.* chevron, star, cross). Preferably the indentations are arranged between each and every longitudinal groove or along each and every rib.

The PSIs comprise a plurality of blunt blades each having a tip **15'** oriented towards the press opening, and arranged in the direction of the press axis. At least one PSI, preferably each and every PSI is spatially separated (*i.e.* isolated) from another PSI in the longitudinal direction. By spatially separated, it is meant that at least the tip **15**', preferably the whole body of a PSI does not contact the tip **15'** of a neighbouring PSI in the longitudinal direction, when the press jaw is closed. Preferably, when the press jaw is closed, the cutting direction of each PSI is orientated radially or inclined to the radius of the press axis, thereby giving rise to radial or radially-inclined indentations in the tampon. The height, DS, of the PSI from its base to the tip (**FIG. 13B**) may be a percentage (fraction) of the height, DL of the PSLG (**FIG. 14B**), which percentage is 10 %, 20 %, 30 %, 40 %, 50 %, 60 %, 70 %, 80 %, or 90 % or a value in the range between any two of the aforementioned values. With reference to **FIGs. 14A** to **C** which depicts a press jaw **6** in side (**14A**), front (**14B**) and plan (**14C**) views, the PSIs **11** are provided on the pressing end of the press jaw **6** and are comprised in a plurality protruding chevrons (**FIG. 14C**) spatially separated in the longitudinal direction of the jaw. Preferably each PSI has the same shape (e.g. chevron, star, cross). Preferably the PSIs are arranged between each and every PSLG.

**FIG. 15A** shows the plan view of the pressing end a press jaw **6**, provided with a plurality of chevron shaped PSIs **11** spatially separated in the longitudinal direction of the jaw **6**. **FIGs. 15B** to **15D** depict numerous view of a finished tampon **220** having a rounded insertion end **230** and a withdrawal end **232**, pressed using a press comprising the press jaw depicted in **FIG. 15A****.** It has four longitudinal grooves **222**, formed by the PSLGs (not shown), and a plurality of chevron shaped indentations **224** spatially separated in the longitudinal direction of the tampon **220** and from the longitudinal grooves **220**.

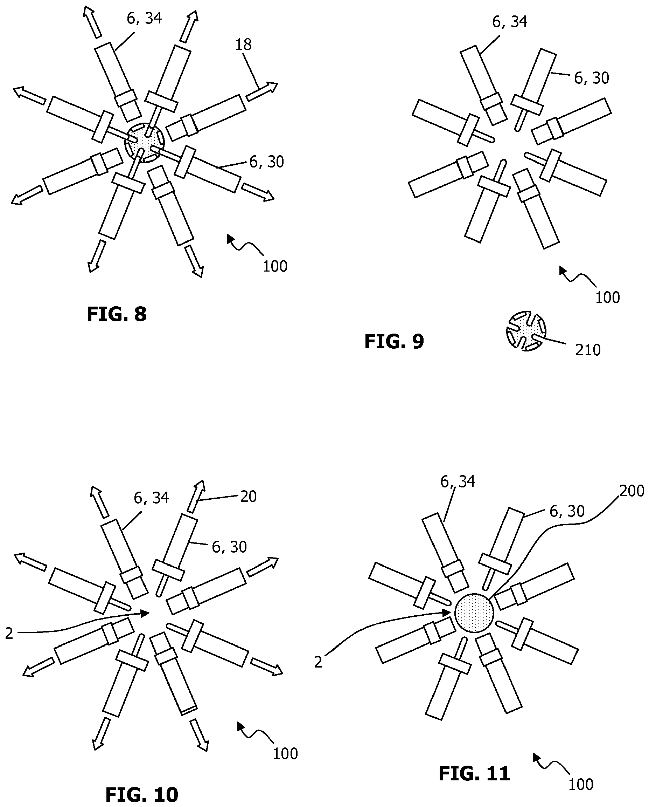

An embodiment of a press apparatus **100** of the current invention and the method is described in more detail below and exemplified by **FIGs. 4 to 11****.** **FIG. 4** shows the press jaws of an embodiment of press **100** according to the invention in open position. **FIG. 4** shows the press jaws of an embodiment of press **100** according to the invention in open position. There are two groups of jaws **6,** the first group **30, 30', 30", 30'''** provided with PSLGs 32, 32', 32", 32''', the second group **34, 34', 34", 34‴,** provided with PSIs **36, 36', 36", 36'''** facing the press opening **2.** The first **30, 30', 30", 30'''** and second group **34, 34', 34", 34'''** of jaws alternate. The press jaws **6** are arranged in a star formation around the press axis **4.** The PSLGs in profile are shown to have a greater maximum height (DL) compared with that of the PSI (DS). **FIG. 4** also shows the press **100** with a blank tampon **200** inserted in the press opening **2.** The press jaws **6** synchronously advance **16** radially towards the press axis **4** (**FIG. 5**), penetrating and pressing the tampon blank **200** as depicted in **FIG. 6****.** While the **FIG. 6** does not show the jaws supported on one another on their mutually opposite longitudinal sides in the closed position, this aspect is preferable and within the scope of the invention. In **FIG. 8** the press jaws are retracted **18** to a holding position, to the extent that the PSI **11** are entirely withdrawn from the preform **210** so produced, in particular from the indentations **214**, while the PSLG **13** remain in contact with the longitudinal grooves **212.** In that holding position, the preform **210** is ejected **(****FIG. 9**) by means of a ram (not shown) that slidably propels the preform along the tips of the PSLG **13**, parallel to the press axis **4** and out towards the other side of the press **100.** The press jaws fully retract **20** (**FIG. 10**), sufficient to allow insertion of a new tampon blank **200** (**FIG. 11**). The grooves **212, 214** in the preform **210** have been enlarged for clarity in the drawings; in practice, the grooves are closed after pressing.

By utilising a differential withdrawal of the PSLG compared with the PSI in the holding position, advantageously, the tampon is suspended circumferentially with minimum contact with the press and is ejected in a slidable manner, guided by the grooves. This results in the ribs and indentations being untouched by the press during ejection, so maintaining their integrity and limiting exposure to contaminants. The tampon can be ejected at great speed. This compares with the art which must allow the tampon to fall to the floor of the press opening. The latter exposes the preform ribs potentially to contamination by the contact with the longitudinal sides of the opened jaw, and to damage by propulsion across a plurality of penetrating segment.

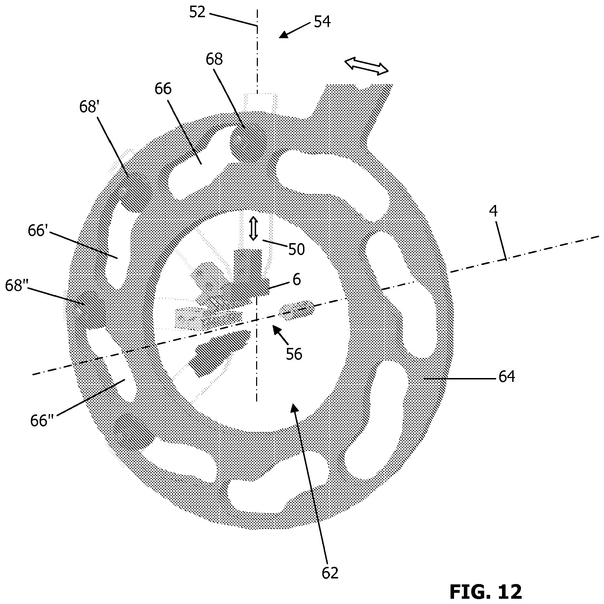

According to one aspect of the invention, as shown in **FIG. 12**, each press jaw **6** is connected to a longitudinal transmission rod **50**, aligned essentially radially to the press axis **4**, or inclined to the radius centred on the press axis. The transmission rod has, at one longitudinal side, a proximal end **56** closer to the press axis **4** and, at the opposing longitudinal side, a distal end **54** directed away from the press axis **4**. The press comprises eight jaws **6**, each in rigid connection with the rod **60.** The rods are configured for slidable linear displacement along an axis of movement **52** that is essentially radial to the press axis **4**, or inclined to the radius. Linear force applied to the distal end **54** of the rod **50** is transmitted to the press jaw **6** which is linearly displaced accordingly. A rotatable annular plate **64** having essentially an annulus shape, disposed with a central part (opening) **62** and having a central axis in co-axial alignment with press axis **4** is provided with a plurality of discrete slots **66** on the plate **64**, one slot for each rod, which slot engages with a roller **68** in revolute attachment to the distal end **54** of each rod **50**. The roller **68** is in slidable connection with the slot **66**. The opening **62** accommodates the press jaws **6**. The axis of rotation of the roller **68** is perpendicular to the longitudinal axis of the rod **52** and is parallel with the press axis **4**. The rotation of the annular plate **64** effects movement of the roller **68** along the axis of movement 52, and translation of the rod **50** towards or away from the press axis **4**. The slot **66** is shaped to retract or advance press jaw **6** in the direction of the press axis **4** according to the angle of rotation of the annular plate **64** around its central axis. The circumferential path of the slot **66** is, at one extreme (preferably end) radially closer to the press axis **4** to obtain a closed press jaw **6**, and at the other extreme (preferably end), radially further removed from the press axis **4** to obtain an open press jaw **6**, the radial distance of the slot **66** path from the central axis transitioning gradually between the extremes. The holding position is maintained by pausing rotation of the annular plate **64** in the transition part of the slot **66**. Rotation of the annular plate **64** around its central axis thereby controls advancement or retraction of the press jaws **6** simultaneously. A production cycle will generally imply consecutive clockwise and anticlockwise rotations of the annular plate **64**, and pausing at the holding position during ejection.

The press jaws can preferably be heated and preferably each press jaw has its own temperature sensor. By heating the press jaws, it is possible to reduce the memory effect of modern, highly absorbent, greatly expanding fibrous materials, which occurs after the tampon has been finished. By means of the heated press jaws, and especially the heated pressing shoulders, the surface of the tampon is simultaneously smoothed during pressing and pushing out, and a qualitatively improved surface is produced in the preformed tampon even in tampon preforms of low weight, the stability of the tampon preform being preserved. The memory effect of the fibrous material becomes effective again when the fibrous material of the tampon is wetted with body fluid.

According to one aspect of the invention, the tips **15, 15'** of the penetrating segments **11, 13** touch a fictive cylinder (PSFC) centred on the press axis when the jaws **6** are in the closed position, and the diameter of the PSFC is constant along the longitudinal axis of the press. According to another aspect of the invention, the PSFC has a variable diameter along the longitudinal axis of the press.

According to one aspect of the invention, the shoulders of the press jaws touch a fictive circle (SFC) centred on the press axis when the jaws **6** are in the closed position, and the diameter of the SFC is constant along the longitudinal axis of the press. According to another aspect of the invention, the SFC has a variable diameter along the longitudinal axis of the press. The variation in SFC longitudinally may provide tampons having different profiles, for example, having a mushroom-shape, domed head, constricted, preferably conical withdrawal end, barrel shape, bullet shape etc.

The invention further concerns a method for manufacturing the tampon in particular for feminine hygiene having a longitudinal body in an essentially cylindrical shape. The tampon is divided into a number of longitudinal grooves that flank longitudinal ribs, and is provided with a plurality of indentations that are spatially arranged in the longitudinal direction. The indentations are preferably spatially separated (isolated) from each other and/or from the longitudinal grooves. A strip of absorbent material having acceptable absorbency and modulus of elasticity properties that is capable of absorbing and/or retaining liquid, is wound up on itself to form an essentially cylindrical tampon blank that is subsequently pressed.

Absorbent fibrous material usable in the tampon produced to the invention may consist of any absorbent material having acceptable absorbency and modulus of elasticity properties that is capable of absorbing and/or retaining liquid. The absorbent structure can be manufactured in a wide variety of sizes and shapes and from a wide variety of liquid-absorbing materials. It is, of course, desirable to use absorbent materials having a minimum content of extraneous soluble materials since the product may be retained in the body for a considerable period of time. Retained soluble extraneous materials could cause a safety hazard if they are toxic, irritant, or sensitive. A representative, non-limiting list of useful materials includes cellulosic materials, such as rayon, cotton, wood pulp, creped cellulose wadding, tissue wraps and laminates, peat moss, and chemically stiffened, modified, or cross-linked cellulosic fibres; synthetic materials, such as polyester fibres, polyolefin fibres, absorbent foams, e.g. a flexible resilient polyurethane foam, absorbent sponges, super-absorbent polymers, absorbent gelling materials; formed fibres, such as capillary channel fibres and multi limbed fibres; synthetic fibres, or any equivalent material or combinations of materials, or mixtures of these.

In one embodiment, the essentially cylindrical blank is not surrounded by a covering, particularly when the blank tampon is made from cotton. In a preferred embodiment, the essentially cylindrical blank is at least partially surrounded by a covering. The covering is preferably not provided at the portion which will form the insertion end of the tampon. In order to improve the absorbing capacity and expansion capacity of the tampon, said covering is preferably a stretchable or elastic liquid-permeable covering.

The tampon blank is pressed with the pressing apparatus described above. In order to form the ribs and indentations of the tampon, the method comprises compressing the tampon blank on its outer circumferential surface, forming longitudinal grooves, indentations and a fibre core. Preferably, the fibre core has a higher degree of compression from which less compressed longitudinal ribs extend outward.

In detail, a preferably cylindrical tampon blank is introduced in the press apparatus described above. The tampon blank is radially compressed or compressed in a direction inclined to the radius by press jaws, such as those described above. If the penetrating segments and the pressing shoulders are fixed to separate press jaws, the tampon blank may be first pressed with the penetrating segments and subsequently with the pressing shoulders. Alternatively, the penetrating segments and the pressing shoulders may press the tampon blank simultaneously. The latter will obviously be the case when the penetrating segments and pressing shoulders are fixed to the same press jaws. In the press, the tampon blank is preferably compressed in a single pressing operation by the penetrating segments and pressing shoulders simultaneously.

The penetrating segments configured to provide longitudinal grooves (PSLG) will preferably press the tampon blank on strips of the circumferential surface which are narrower than the strips of the circumferential surface pressed by the pressing shoulders. Preferably also, the strips pressed by the penetrating segments have an equal length and width and the strips pressed by the pressing shoulders also have an equal length and width. In this way, ribs are formed, defined by longitudinal grooves on a solid fibre core. The penetrating segments configured to provide indentations (PSI) press the tampon blank on the ribs, between the grooves. The pressing shoulders will press on the circumference of the so formed ribs and indentations in order to obtain an essentially cylindrical form with a smaller diameter. The memory effect of the tampon blank maintains the shape of the compressed tampon form.

The tampon blank, having been pressed by the penetrating segments and pressing shoulders, forms a preform which is ejected from the press. Prior to ejection, the press jaws retract simultaneously to the extent that the PSIs are fully withdrawn from the preform, while contact is maintained between the preform and the PSLGs. Thus, the preform becomes suspended in the partially closed jaws by the circumferential arrangement of PSLGs, but is free from contact with the PSI. Accordingly, the preform is propelled longitudinally while in slidable contact only with the PSLGs. By invoking a slidable ejection along the PSLGs, the integrity of the preform is maintained, avoiding damage to its surface by otherwise protruding PSIs in the ejection passage. Equally, ejection of the preform while the jaws are open fully would lead to damage as the space between two jaws into which the tampon is released provides only an obstructive and unhygienic passage for slidable ejection. An example of a preform formed by the press of the invention is schematically depicted in **FIG. 9****.**

One embodiment of the invention concerns a process for manufacturing a tampon having a longitudinal axis, comprising the steps:
a) inserting a cylindrical blank **200** of absorbing material in a press for manufacturing a tampon which presses absorbing material radially or inclined to the radius, which press comprises at least three press jaws **6** arranged in a star formation, whereby there is provided on a single or separate adjacent press jaws:
   - a penetrating segment, PSLG, configured to penetrate the absorbing material with a longitudinal groove,
   - penetrating segments, PSI, configured to penetrate the absorbing material with a plurality of indentations that are arranged in the longitudinal direction,
b) pressing the tampon blank in the press jaws, such that:
   - the PSLG penetrates the cylindrical blank to form longitudinal ribs 12 defined by longitudinal grooves,
   - PSI penetrate the cylindrical blank to form a plurality of indentations that are arranged in the longitudinal direction, between the longitudinal grooves,
      so forming a preform,
c) moving the press jaws to a holding position between the closed position and open position, so that the preform can be removed, and
d) removing the pressed cylindrical blank from the press while the press jaws are maintained in the holding position.

Besides being arranged in the longitudinal direction, at least one indentation, preferably each and every indentation may also be spatially separated from other or adjacent side grooves. The at least one indentation, preferably each and every indentation may also be spatially separated from the adjacent longitudinal grooves. In step b) the press jaws **6** are moved (advanced) to a closed position to press the cylindrical blank **200** so forming a preform **210** (pressed cylindrical blank). The movement, towards the press axis, is preferably radial or may be inclined to the radius of the press axis. The cylindrical blank is, thus, pressed radially or may be pressed inclined to the radius of the press axis.
In step c), the press jaws **6** are moved (retracted) to a holding position between the closed position and open position, so that the preform can be removed axially without substantial obstruction. Preferably, the holding position is where the tips **15'** of the PSIs **11** are moved (retracted) to a greater distance from the press axis **4** compared with the tips **16** of the PSLGs, such that the PSIs are withdrawn, preferably fully, from the preform, while contact is maintained between the preform longitudinal grooves and the PSLGs **13.**
In step d) the preform is removed (ejected), preferably slidably, while the jaws (6) are in the holding position. In a subsequent step, the press jaws **6** are moved (retracted) to the open position for loading of another cylindrical blank. The sequence of steps may be repeated for a subsequent cylindrical blank.

The press used in the process may that as defined elsewhere herein.

Another embodiment of the invention is a tampon obtainable by a process of the invention.

This preform may be simultaneously subjected to final shaping downstream so forming a tampon. This final shaping includes a radial pressure being exerted on the total circumference of the preform. This radial pressure has the effect that the adjacent longitudinal ribs are pressed against one another, so that the longitudinal grooves are substantially closed and the circumferential surface of the tampon is substantially smooth and soft.

The tampon blank is, depending on the properties of the fibrous material used, in particular in the event of use being made of highly expansive fibres of irregular cross section with a strong memory effect, pressed at a temperature of the press jaws to the final shape of the tampon, in order to achieve the desired dimensional stability of the fibrous material by eliminating the memory effect of the fibres, which immediately becomes effective again on contact with bodily fluid and thus increases the expansion and absorption speed of the tampon with the least possible use of fibrous material.

## Claims

1. A tampon (200) for feminine hygiene having a longitudinal body, whereby said tampon comprises compressed absorbent fibrous material and has an outer circumferential surface which is provided with longitudinal grooves (222) that are separated from each other by longitudinal ribs (226), **characterised in that** the number of longitudinal ribs (226) is at least four; that the tampon further comprises a plurality of discrete indentations (224) arranged in the longitudinal direction along a longitudinal rib (226) and flanked by two longitudinal grooves; that at least one indentation (224) is in spatial isolation from one or both flanking longitudinal grooves (222); and **in that** at least one indentation (224) has the shape essentially of a chevron (v-shaped), undulating, star, cross, diamond, triangle, pentagon, sexagon, septagon, octagon, nonagon, or decagon, wherein the longitudinal grooves (222) are straight, the longitudinal grooves (222) extending fully from an insertion end to a withdrawal end.

2. Tampon (200) according to claim 1, having a mushroom-shape, domed head, constricted withdrawal end, conical withdrawal end, barrel shape, rivet shape, finger recess, withdrawal cord or a bullet shape.

3. Tampon (200) according to any of claims 1 to 2, disposed in an applicator.

4. Tampon (200) according to any of claims 1 to 3, provided with one or more markings on the surface.

5. Tampon (200) according to any of claims 1 to 4, wherein the indentations and/or the longitudinal grooves are radial or radially-inclined.

6. Tampon (200) according to any of claims 1 to 5, wherein at least one indentation (224) is spatially separated from other adjacent indentations.

7. Press (100) for manufacturing a tampon according to any of claims 1 to 6, comprising at least four press jaws (6) arranged in a star around a central longitudinal press axis (4) forming a press opening (2), whereby there is provided on a single or separate adjacent press jaws (6):
- a penetrating segment, PSLG, (13) configured to penetrate the absorbing material with a longitudinal groove, and
- penetrating segments, PSI, (11) configured to penetrate the absorbing material with a plurality of indentations that are arranged in the longitudinal direction, at least one indentation being spatially separated from the longitudinal groove,
wherein the press is configured to:
a) load a cylindrical blank (200) in the press opening (2),
b) move the press jaws (6) to a closed position to press the cylindrical blank (200) so forming a preform (210),
**characterized in that** the press is further configured to:
c) move the press jaws (6) to a holding position between the closed position and open position, so that the preform can be removed,
d) remove the preform while the jaws (6) are in the holding position, and
e) move the press jaws (6) to the open position for loading of a subsequent cylindrical blank.

8. Press according to claim 7, where the press jaws are configured to move synchronously.

9. Press according to claim 7 or 8, wherein at least one, preferably each and every PSI is in the shape of a chevron (v-shaped), straight-edged slot, undulating, star, cross, diamond, circular, oval, triangle, rectangle, pentagon, sexagon, septagon, octagon, nonagon, decagon, or other polygon.

10. Press according to any of claims 7 to 9, wherein the number of PSSGs disposed on a press jaw is between 3 and 7.

11. Press according to any of claims 7 to 10, wherein the maximum height, DL, of the PSLG from the base (17) to the tip (15) is greater than the maximum height, DS, of the PSI from the base (17) to the tip (15).

12. Press according to any of claims 7 to 11, wherein the press jaws further comprise one or more pressing shoulders for finish shaping of the preform.

13. Press according to any of claims 7 to 12, wherein during pressing, the pressing shoulders are configured to produce a preform having a mushroom-shape, domed head, constricted withdrawal end, conical withdrawal end, barrel shape, rivet or a bullet shape.

14. Press according to any of claims 7 to 13, wherein:
- each press jaw (6) is connected to a longitudinal transmission rod (50), aligned essentially radially to the press axis (4), or inclined to the radius centred on the press axis, said rod having a proximal end (56) closer to the press axis (4) and, at the opposing longitudinal side, a distal end (54) directed away from the press axis (4), and is configured for slidable linear displacement along an axis of movement (52) that is essentially radial to the press axis (4), or inclined to said radius.
- the press further comprises a rotatable annular plate (64) having a central axis in co-axial alignment with press axis (4) and provided with a plurality of discrete slots (66) on the plate (64), one slot for each rod, which slot engages with a roller (68) in revolute attachment to the distal end (54) of each rod (50), the roller (68) being in slidable connection with the slot (66), the axis of rotation of the roller (68) being perpendicular to the longitudinal axis of the rod (52) and is parallel with the press axis (4),
- the ring (60) is configured to rotate and thereby effect movement of the roller (68) and translation the rod (50) towards or away from the press axis (4) along the axis of movement (52), and
- the slot (66) shaped to retract or advance each press jaw (6) in the direction of the press axis (4) according to the angle of rotation of the annular plate (64) around its central axis.

15. Press according to any of claims 7 to 14, wherein the press jaws (6) in step b) are moved to a closed position in a direction essentially radial or inclined to the radius of the press axis (4).

16. Press according to any of claims 7 to 15, wherein the press jaws (6) in step c) are moved to a holding position between the closed position and open position, in which the tips (15') of the PSIs (11) are retracted to a greater distance from the press axis (4) compared with the tips (15) of the PSLGs, such that the PSIs are fully withdrawn from the preform, while contact is maintained between the preform longitudinal grooves and at least one PSLG (13).

17. Process for manufacturing a tampon according to any of claims 1 to 6 having a longitudinal axis, comprising the steps:
- inserting a cylindrical blank (200) of absorbing material in a press for manufacturing a tampon which presses absorbing material radially, which press comprises at least four press jaws (6) arranged in a star formation, whereby there is provided on a single or separate adjacent press jaws:
- a penetrating segment, PSLG, configured to penetrate the absorbing material with a longitudinal groove, and
- penetrating segments, PSIs, configured to penetrate the absorbing material with a plurality of indentations that are arranged in the longitudinal direction, at least one indentation being spatially separated from the longitudinal groove,
- pressing the tampon blank in the press jaws, such that:
- the PSLG penetrates the cylindrical blank to form longitudinal ribs (12) defined by longitudinal grooves,
- PSIs penetrate the cylindrical blank to form a plurality of indentations that are spatially arranged the longitudinal direction, at least one indentation being spatially separated from adjacent indentations and longitudinal grooves,
so forming a preform,
**characterized in that** the process further comprises the steps:
- moving the press jaws to a holding position between the closed position and open position, so that the preform can be removed,
- removing the preform from the press while the press jaws are maintained in the holding position.

18. Process according to claim 17, wherein the press is as defined in any of claims 7 to 16.

## Patentansprüche

1. Tampon (200) für Damenhygiene, der einen länglichen Körper aufweist, wobei der Tampon komprimiertes absorbierendes faserförmiges Material umfasst und eine außen umlaufende Oberfläche aufweist, die mit Längsrillen (222) versehen ist, die voneinander durch Längsrippen (226) getrennt sind, **dadurch gekennzeichnet, dass** die Anzahl der Längsrippen (226) mindestens vier beträgt, dass der Tampon ferner mehrere einzelne Einprägungen (224) umfasst, die in der Längsrichtung entlang einer Längsrippe (226) angeordnet und von zwei Längsrillen flankiert sind, dass mindestens eine Einprägung (224) räumlich von einer oder beiden flankierenden Längsrillen (222) isoliert ist, und dadurch, dass mindestens eine Einprägung (224) im Wesentlichen eine Winkelform (V-Form), Wellen-, Stern-, Kreuz-, Rauten-, Dreieck-, Fünfeck-, Sechseck-, Siebeneck-, Achteck-, Neuneck- oder Zehneckform aufweist, wobei die Längsrillen (222) gerade sind und wobei sich die Längsrillen (222) vollständig von einem Einführende bis zu einem Entnahmeende erstrecken.

2. Tampon (200) nach Anspruch 1, eine Pilzform, einen gewölbten Kopf, ein eingeengtes Entnahmeende, ein konisches Entnahmeende, eine Tonnenform, eine Nietenform, eine Fingervertiefung, ein Entnahmeband oder eine Projektilform aufweisend.

3. Tampon (200) nach einem der Ansprüche 1 bis 2, angeordnet in einem Applikator.

4. Tampon (200) nach einem der Ansprüche 1 bis 3, auf der Oberfläche mit einer oder mehreren Markierungen versehen.

5. Tampon (200) nach einem der Ansprüche 1 bis 4, wobei die Einprägungen und/oder die Längsrillen radial oder radial-schräg sind.

6. Tampon (200) nach einem der Ansprüche 1 bis 5, wobei mindestens eine Einprägung (224) räumlich von anderen benachbarten Einprägungen getrennt ist.

7. Presse (100) zum Herstellen eines Tampons nach einem der Ansprüche 1 bis 6, mindestens vier Pressbacken (6) umfassend, die sternförmig um eine mittlere längsgerichtete Pressachse (4) angeordnet sind, welche eine Pressöffnung (2) bildet, wobei an einer einzelnen oder getrennten benachbarten Pressbacke (6) Folgendes bereitgestellt ist:
- ein penetrierendes Segment, PSLG, (13), das dafür gestaltet ist, das absorbierende Material mit einer Längsrille zu penetrieren, und
- penetrierende Segmente, PSI, (11), die dafür gestaltet sind, das absorbierende Material mit mehreren Einprägungen zu penetrieren, die in der Längsrichtung angeordnet sind, wobei mindestens eine Einprägung von der Längsrille räumlich getrennt ist,
wobei die Presse für Folgendes gestaltet ist:
a) Laden eines zylinderförmigen Rohlings (200) in die Pressöffnung (2),
b) Bewegen der Pressbacken (6) in eine geschlossene Position, um den zylinderförmigen Rohling (200) zu pressen und so eine Vorform (210) zu bilden, **dadurch gekennzeichnet, dass** die Presse ferner für Folgendes gestaltet ist:
c) Bewegen der Pressbacken (6) zu einer Halteposition zwischen der geschlossenen Position und der offenen Position, so dass die Vorform entfernt werden kann,
d) Entfernen der Vorform, während sich die Pressbacken (6) in der Halteposition befinden, und
e) Bewegen der Pressbacken (6) in die offene Position, um einen folgenden zylinderförmigen Rohling zu laden.

8. Presse nach Anspruch 7, wobei die Pressbacken dafür gestaltet sind, sich synchron zu bewegen.

9. Presse nach Anspruch 7 oder 8, wobei mindestens ein, vorzugsweise jedes PSI in einer Winkelform (V-Form), in Form eines geradkantigen Schlitzes, einer Wellen-, Stern-, Kreuz-, Rauten-, Kreis-, Oval-, Dreieck-, Rechteck-, Fünfeck-, Sechseck-, Siebeneck-, Achteck-, Neuneck- oder Zehneckform oder eines anderen Vielecks vorliegt.

10. Presse nach einem der Ansprüche 7 bis 9, wobei die Anzahl an PSSG, die an einer Pressbacke angeordnet sind, zwischen 3 und 7 beträgt.

11. Presse nach einem der Ansprüche 7 bis 10, wobei die maximale Höhe, DL, des PSLG vom Fuß (17) bis zur Spitze (15) größer als die maximale Höhe, DS, des PSI vom Fuß (17) bis zur Spitze (15) ist.

12. Presse nach einem der Ansprüche 7 bis 11, wobei die Pressbacken ferner eine oder mehrere Pressansätze umfassen, um das Formen der Vorform abzuschließen.

13. Presse nach einem der Ansprüche 7 bis 12, wobei die Pressansätze dafür gestaltet sind, während des Pressens eine Vorform zu produzieren, die eine Pilzform, einen gewölbten Kopf, ein eingeengtes Entnahmeende, ein konisches Entnahmeende, eine Tonnenform, eine Nietenform oder eine Projektilform aufweist.

14. Presse nach einem der Ansprüche 7 bis 13, wobei:
- jede Pressbacke (6) mit einer längsgerichteten Getriebestange (50) verbunden ist, die im Wesentlichen radial zur Pressachse (4) ausgerichtet oder zum an der Pressachse vermittelten Radius schräg liegt, wobei die Stange ein nahes Ende (56) aufweist, das näher zur Pressachse (4) liegt, und an der gegenüberliegenden Längsseite ein fernes Ende (54), das weg von der Pressachse (4) gerichtet ist, und die Stange für eine gleitende lineare Verschiebung entlang einer Bewegungsachse (52), die im Wesentlichen radial zur Pressachse (4) oder schräg zu dem Radius verläuft, gestaltet ist,
- die Presse ferner eine drehbare ringförmige Platte (64) umfasst, die eine Mittelachse in koaxialer Ausrichtung mit der Pressachse (4) umfasst und mit mehreren einzelnen Schlitzen (66) in der Platte (64) versehen ist, ein Schlitz für jede Stange, wobei der Schlitz mit einer rollenden Anlagerung an dem fernen Ende (54) jeder Stange (50) mit einer Walze (68) in Eingriff steht, wobei die Walze (68) in gleitender Verbindung mit dem Schlitz (66) steht, wobei die Drehachse der Walze (68) senkrecht zur Längsachse der Stange (52) und parallel zur Pressachse (4) verläuft,
- der Ring (60) dafür gestaltet ist, sich zu drehen und dadurch eine Bewegung der Walze (68) und eine Verschiebung der Stange (50) hin zu oder weg von der Pressachse (4) entlang der Bewegungsachse (52) zu bewirken, und
- der Schlitz (66) dafür geformt ist, jede Pressbacke (6) gemäß dem Drehwinkel der ringförmigen Plate (64) um ihre Mittelachse in der Richtung der Pressachse (4) zurückzuziehen oder vorzuschieben.

15. Presse nach einem der Ansprüche 7 bis 14, wobei die Pressbacken (6) in Schritt b) in eine Richtung, die im Wesentlichen radial oder schräg zum Radius der Pressachse (4) verläuft, zu einer geschlossenen Position bewegt werden.

16. Presse nach einem der Ansprüche 7 bis 15, wobei die Pressbacken (6) in Schritt c) zu einer Halteposition zwischen der geschlossenen Position und der offenen Position bewegt werden, in welcher die Spitzen (15') der PSI (11) in eine größere Entfernung zur Pressachse (4) zurückgezogen sind, als die Spitzen (15) der PSLG, so dass die PSI vollständig von der Vorform abgezogen sind, während der Kontakt zwischen den Längsrillen der Vorform und mindestens einem PSLG (13) aufrechterhalten ist.

17. Verfahren zum Herstellen eines Tampons nach einem der Ansprüche 1 bis 6, der eine Längsachse aufweist, Folgende Schritte umfassend:
- Einsetzen eines zylinderförmigen Rohlings (200) aus absorbierendem Material in eine Presse zum Herstellen eines Tampons, die das absorbierende Material radial presst, wobei die Presse mindestens vier Pressbacken (6) umfasst, die sternförmig angeordnet sind, wobei an einer einzelnen oder getrennten benachbarten Pressbacke (6) Folgendes bereitgestellt ist:
- ein penetrierendes Segment, PSLG, (32, 32', 32", 32‴), das dafür gestaltet ist, das absorbierende Material mit einer Längsrille zu penetrieren, und
- penetrierende Segmente, PSI (36, 36', 36", 36‴), die dafür gestaltet sind, das absorbierende Material mit mehreren Einprägungen zu penetrieren, die in der Längsrichtung angeordnet sind, wobei mindestens eine Einprägung von der Längsrille räumlich getrennt ist,
Pressen des Tamponrohlings in den Pressbacken (6) derart, dass:
- die PSLG (32, 32', 32", 32‴) den zylinderförmigen Rohling penetrieren, um Längsrippen (12) zu bilden, die durch Längsrillen definiert sind,
- die PSI (36, 36', 36", 36‴) den zylinderförmigen Rohling penetrieren, um mehrere Einprägungen zu bilden, die räumlich in der Längsrichtung angeordnet sind, wobei mindestens eine Einprägung räumlich von benachbarten Einprägungen und Längsrillen getrennt ist,
so dass eine Vorform gebildet wird,
**dadurch gekennzeichnet, dass** das Verfahren ferner folgende Schritte umfasst:
- Bewegen (18) der Pressbacken (6) zu einer Halteposition zwischen der geschlossenen Position und der offenen Position, so dass die Vorform (210) entfernt werden kann,
- Entfernen der Vorform (210) aus der Presse, während die Pressbacken (6) in der Halteposition gehalten werden.

18. Prozess nach Anspruch 17, wobei die Presse ist, wie in einem der Ansprüche 7 bis 16 definiert.

## Revendications

1. Tampon (200) d'hygiène féminine ayant un corps longitudinal, moyennant quoi ledit tampon comprend un matériau fibreux absorbant compressé et possède une surface circonférentielle externe qui est munie de rainures longitudinales (222) qui sont séparées les unes des autres par des nervures longitudinales (226), **caractérisé en ce que** le nombre de nervures longitudinales (226) est au moins de quatre ; **en ce que** le tampon comprend en outre une pluralité d'indentations discrètes (224) prévues dans la direction longitudinale le long d'une nervure longitudinale (226) et entourées de deux rainures longitudinales ; **en ce qu'**au moins une indentation (224) est isolée spatialement d'une ou des deux rainures longitudinales situées autour (222) ; et **en ce qu'**au moins une indentation (224) possède en fait la forme d'un chevron (forme de v), d'une ondulation, d'une étoile, d'une croix, d'un diamant, d'un triangle, d'un pentagone, d'un hexagone, d'un heptagone, d'un octogone, d'un ennéagone, ou d'un décagone, et dans lequel les rainures longitudinales (222) sont droites, les rainures longitudinales (222) s'étendant entièrement d'une extrémité d'insertion à une extrémité de retrait.

2. Tampon (200) selon la revendication 1, ayant une forme de champignon, une tête en dôme, une extrémité de retrait resserrée, une extrémité de retrait conique, une forme de barillet, une forme de rivet, un renfoncement pour le doigt, un cordon de retrait ou une forme de balle.

3. Tampon (200) selon l'une quelconque des revendications 1 à 2, disposé dans un applicateur.

4. Tampon (200) selon l'une quelconque des revendications 1 à 3, muni d'un ou plusieurs repère(s) sur la surface.

5. Tampon (200) selon l'une quelconque des revendications 1 à 4, dans lequel les indentations et/ou les rainures longitudinales sont radiales ou inclinées radialement.

6. Tampon (200) selon l'une quelconque des revendications 1 à 5, dans lequel au moins une indentation (224) est spatialement séparée des autres indentations adjacentes.

7. Presse (100) de fabrication d'un tampon selon la revendication 1 à 6, comprenant au moins quatre mâchoires de presse (6) disposées en étoile autour d'un axe de presse longitudinal central (4) en formant une ouverture de presse (2), moyennant quoi est prévu sur une seule mâchoire de presse ou sur les mâchoires de pression adjacentes distinctes (6) :
- un segment de pénétration, PSLG, (13) configuré pour pénétrer dans le matériau absorbant avec une rainure longitudinale, et
- des segments de pénétration, PSI, (11) configurés pour pénétrer dans le matériau absorbant avec une pluralité d'indentations qui sont disposées dans la direction longitudinale, au moins une indentation étant spatialement séparée de la rainure longitudinale,
dans laquelle la presse est configurée pour :
a) charger une ébauche cylindrique (200) dans l'ouverture de presse (2),
b) déplacer les mâchoires de presse (6) dans une position fermée afin de presser l'ébauche cylindrique (200) de façon à former une préforme (210),
**caractérisée en ce que** la presse est en outre configurée pour :
c) déplacer les mâchoires de la presse (6) dans une position de maintien entre la position fermée et la position ouverte, de sorte que la préforme puisse être retirée,
d) retirer la préforme pendant que les mâchoires (6) se trouvent en position de maintien, et
e) déplacer les mâchoires de la presse (6) en position ouverte afin de charger une ébauche cylindrique suivante.

8. Presse selon la revendication 7, dans laquelle les mâchoires de la presse sont configurées pour se déplacer de manière synchrone.

9. Presse selon la revendication 7 ou 8, dans laquelle au moins un, de préférence chaque PSI, possède la forme d'un chevron (forme de v), d'une fente à bord droit, d'une ondulation, d'une étoile, d'une croix, d'un diamant, d'un cercle, d'un ovale, d'un triangle, d'un rectangle, d'un pentagone, d'un hexagone, d'un heptagone, d'un octogone, d'un ennéagone, d'un décagone, ou d'un autre polygone.

10. Presse selon l'une quelconque des revendications 7 à 9, dans laquelle le nombre de PSSG disposés sur une mâchoire de presse est compris entre 3 et 7.

11. Presse selon l'une quelconque des revendications 7 à 10, dans laquelle la hauteur maximale, DL, du PSLG entre la base (17) et l'extrémité (15) est supérieure à la hauteur maximale, DS, du PSI entre la base (17) et l'extrémité (15).

12. Presse selon l'une quelconque des revendications 7 à 11, dans laquelle les mâchoires de la presse comprennent en outre un ou plusieurs épaulement(s) de pressage destiné(s) à la finition de mise en forme de la préforme.

13. Presse selon l'une quelconque des revendications 7 à 12, dans laquelle, pendant le pressage, le épaulements de pressage sont configurés pour produire une préforme ayant une forme de champignon, une tête en dôme, une extrémité de retrait resserrée, une extrémité de retrait conique, une forme de barillet, une forme de rivet ou une forme de balle.

14. Presse selon l'une quelconque des revendications 7 à 13, dans laquelle :
- chaque mâchoire de presse (6) est reliée à une tige de transmission longitudinale (50), alignée de manière essentiellement radiale avec l'axe de presse (4), ou inclinée par rapport au rayon centré sur l'axe de presse, ladite tige ayant une extrémité proximale (56) plus proche de l'axe de presse (4) et, au niveau du côté longitudinal opposé, une extrémité distale (54) éloignée de l'axe de presse (4), et est configurée pour se déplacer linéairement de manière coulissante le long d'un axe de déplacement (52) qui est essentiellement radial par rapport à l'axe de presse (4), ou incliné par rapport audit rayon,
- la presse comprend en outre une plaque annulaire rotative (64) ayant un axe central en alignement coaxial avec l'axe de presse (4) et munie d'une pluralité de fentes discrètes (66) sur la plaque(64), une fente pour chaque tige, ladite fente s'engageant avec un galet (68) relié de manière pivotante à l'extrémité distale (54) de chaque tige (50), le galet (68) étant relié de manière coulissante à la fente (66), l'axe de rotation du galet (68) étant perpendiculaire à l'axe longitudinal de la tige (52) et parallèle à l'axe de presse (4),
- la bague (60) est configurée pour tourner et ainsi provoquer le mouvement du galet (68) et la translation de la tige (50) vers ou à l'écart de l'axe de presse (4) le long de l'axe de déplacement (52), et
- la fente (66) est formée pour rétracter ou faire avancer chaque mâchoire de presse (6) dans la direction de l'axe de presse (4) selon l'angle de rotation de la plaque annulaire (64) autour de son axe central.

15. Presse selon l'une quelconque des revendications 7 à 14, dans laquelle les mâchoires de presse (6) à l'étape b) sont déplacées dans une position fermée dans une direction essentiellement radiale ou inclinée par rapport au rayon de l'axe de presse (4).

16. Presse selon l'une quelconque des revendications 7 à 15, dans laquelle les mâchoires de presse (6) à l'étape c) sont déplacées dans une position de maintien entre la position fermée et la position ouverte, dans laquelle les extrémités (15') des PSI (11) sont rétractées sur une plus grande distance par rapport à l'axe de presse (4) en comparaison avec les extrémités (15) des PSLG, de sorte que les PSI soient entièrement retirés de la préforme, tandis qu'un contact est maintenu entre les rainures longitudinales de la préforme et au moins un PSLG (13).

17. Procédé de fabrication d'un tampon selon l'une quelconque des revendications 1 à 6 ayant un axe longitudinal, comprenant les étapes consistant à :
- insérer une ébauche cylindrique (200) de matériau absorbant dans une presse de fabrication d'un tampon qui presse le matériau absorbant radialement, ladite presse comprenant au moins quatre mâchoires de presse (6) disposées en étoile, moyennant quoi est prévu, sur une seule mâchoire de presse ou sur les mâchoires de presse adjacentes (6) :
- un segment de pénétration, PSLG (32, 32', 32", 32‴) configuré pour pénétrer dans le matériau absorbant avec une rainure longitudinale, et
- des segments de pénétration, PSI (36, 36', 36", 36''') configurés pour pénétrer dans le matériau absorbant avec une pluralité d'indentations qui sont disposées dans la direction longitudinale, au moins une indentation étant spatialement séparée de la rainure longitudinale,
- le fait de presser l'ébauche de tampon dans les mâchoires de presse (6), de sorte que :
- le PSLG (32, 32', 32'', 32''') pénètre dans l'ébauche cylindrique afin de former les nervures longitudinales (12) définies par les rainures longitudinales,
- les PSI (36, 36', 36", 36''') pénètrent dans l'ébauche cylindrique afin de former une pluralité d'indentations qui sont agencées spatialement dans la direction longitudinale, au moins une indentation étant séparée spatialement des indentations adjacentes et des rainures longitudinales, afin de former une préforme,
**caractérisé en ce que** le procédé comprend en outre les étapes consistant à :
- déplacer (18) les mâchoires de presse (6) dans une position de maintien entre la position fermée et la position ouverte, de sorte que la préforme (210) puisse être retirée,
- retirer la préforme (210) de la presse pendant que les mâchoires de presse (6) sont maintenues dans la position de maintien.

18. Procédé selon la revendication 17, dans lequel la presse est telle que définie selon l'une quelconque des revendications 7 à 16.
